(19) Europäisches Patentamt
European Patent Office
Office européen des brevets

(11) EP 2 407 454 A1

(12) **EUROPEAN PATENT APPLICATION**
published in accordance with Art. 153(4) EPC

(43) Date of publication:
**18.01.2012 Bulletin 2012/03**

(21) Application number: **10750846.7**

(22) Date of filing: **10.03.2010**

(51) Int Cl.:
***C07D 209/42** (2006.01)* ***A61K 31/405** (2006.01)*
***A61P 27/02** (2006.01)* ***C07D 403/06** (2006.01)*

(86) International application number:
**PCT/JP2010/053940**

(87) International publication number:
**WO 2010/104093 (16.09.2010 Gazette 2010/37)**

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **10.03.2009 JP 2009055998**

(71) Applicant: **Santen Pharmaceutical Co., Ltd**
**Osaka-shi**
**Osaka 533-8651 (JP)**

(72) Inventors:
- **SASAOKA, Masaaki**
**Ikoma-shi**
**Nara 630-0101 (JP)**
- **UMEMOTO, Tomoko**
**Ikoma-shi**
**Nara 630-0101 (JP)**
- **SEIKE, Hisayuki**
**Ikoma-shi**
**Nara 630-0101 (JP)**
- **KAGEYAMA, Masaaki**
**Ikoma-shi**
**Nara 630-0101 (JP)**

(74) Representative: **Müller-Boré & Partner Patentanwälte**
**Grafinger Straße 2**
**81671 München (DE)**

(54) **PROPHYLACTIC OR THERAPEUTIC AGENT FOR OPTIC NERVE DISORDERS COMPRISING 4,6-DICHLORO-1H-INDOLE-2-CARBOXYLIC ACID DERIVATIVE OR SALT THEREOF AS ACTIVE INGREDIENT**

(57) Disclosed is a compound useful as a preventive or therapeutic agent for optic nerve disorders, an inhibitor of retinal ganglion cell death or an agent for restoring the expression level of a neurofilament light chain, particularly a compound capable of exhibiting the abovementioned pharmacological activities when administered orally. A 4,6-dichloro-1H-indole-2-carboxylic acid derivative or a salt thereof can significantly inhibit the decrease in the number of cells in a retinal ganglion cell layer of an NMDA-induced rat retinal disorder model when administered to the model orally, and can also restore the decrease in the expression level of a neurofilament light chain in a retina of the model when administered to the model intravitreally (intraocular local administration). Therefore, the compound or a salt thereof is useful as a preventive or therapeutic agent for optic nerve disorders, an inhibitor of retinal ganglion cell death or an agent for restoring the expression level of a neurofilament light chain.

EP 2 407 454 A1

## Description

Technical Field

The present invention relates to a preventive or therapeutic agent for an optic nerve disorder, a retinal nerve cell death inhibitor, or a neurofilament light chain expression level recovering agent comprising at least one of 4,6-dichloro-1H-indole-2-carboxylic acid derivatives or salts thereof as an active ingredient.

Background Art

A retina is a tissue of 0.1 to 0.5 mm in thickness consisting of 10 layers, namely, an inner limiting membrane, a nerve fiber layer, a ganglion cell layer, an inner plexiform layer, an inner nuclear layer, an outer plexiform layer, an outer nuclear layer, an outer limiting membrane, a photoreceptor cell layer and a retinal pigmented epithelial layer, in which there are retinal nerve cells such as a photoreceptor cell, a bipolar cell, a ganglion cell, a horizontal cell and an amacrine cell.

A retinal nerve cell plays an important role in receiving and transmitting a visual information such as converting an optical stimulation into an electric signal which it then transmits to a brain.

In its transmission mechanism, when discussed in detail, a visual information entering an eye is converted by a photoreceptor cell into an electric signal which is transmitted, via a horizontal cell, a bipolar cell and/or an amacrine cell, to a ganglion cell. Then, the electric signal is transmitted, via an optic nerve which is a bundle of optic nerve fibers including a ganglion cell axon, to a brain.

When an optic nerve is impaired by various causes, a homeostasis of the optic nerve can not be maintained, resulting in an interference with the transmission of a visual information to a brain, which leads to a vision disorder such as blindness and constriction of visual fields.

While various causes of an optic nerve impairment are considered, a major cause may for example be 1)a rise of intraocular pressure, 2)a retinal blood circulation impairment/retinal ischemia, 3)an increased excitatory amino acid and the like, and these pathologies are accompanied with an activation of a glutamic acid signal cascade or a retinal ganglion cell axon impairment followed by a retinal nerve cell apoptosis, by which the optic nerve is considered to be impaired.

Accordingly, a drug which protects a retinal nerve cell by blocking a part of the glutamic acid signal cascade whereby inhibiting a retinal nerve cell apoptosis, i.e., a drug such as a glutamic acid neurotoxicity inhibitor, an N-methyl-D-aspartic acid (hereinafter referred to also as "NMDA") receptor blocker, a nitrogen monoxide synthesis inhibiting agent and the like, is considered to be useful as a preventive or therapeutic agent for an optic nerve disorder or an ophthalmic disease accompanying it, and various studies are undertaken currently.

For example, those disclosed are a retinal nerve cell protecting agent comprising as an active ingredient one of β blockers, namely, nipradilol, in Patent Document 1, an optic ganglion cell protecting agent comprising as an active ingredient an interleukin-1 receptor antagonist protein in Patent Document 2, a optic ganglion cell protecting agent comprising as an active ingredient an $\alpha_1$ receptor blocker such as bunazosin in Patent Document 3, a retinal nerve cell protecting agent comprising as an active ingredient a fluorine-containing prostaglandin F2α derivative in Patent Document 4, and a retinal nerve cell protecting agent comprising as an active ingredient an indazole derivative in Patent Document 5.

Representative ophthalmic diseases accompanied with optic nerve disorders may for example be glaucomatous diseases, retinal diseases, ischemic disorders and the like such as glaucoma, glaucomatous constriction of visual fields, glaucomatous optic nerve atrophy, glaucomatous optic neurosis, central retinal artery occlusion, branch retinal arterial occlusion, central retinal vein occlusion, branch retinal venous occlusion, diabetic retinopathy, macular degeneration (age-related macular degeneration such as atrophic age-related macular degeneration, exudative age-related macular degeneration and the like), retinitis pigmentosa, retinopathy of prematurity, retinal detachment, retinal pigment epithelial detachment, Leber disease, ischemic optic neurosis and the like.

On the other hand, sodium (E)-4,6-dichloro-3-(3-oxo-3-(phenylamino)-1-propenyl)-1H-indole-2-carboxylate (GV-150526A) which is one of 4,6-dichloro-1H-indole-2-carboxylic acid derivatives or salts thereof is described in Patent Document 1 and Patent Document 2 as an excitatory amino acid antagonistic agent useful as a therapeutic agent for Alzheimer disease, Huntington disease, amyotrophic lateral sclerosis and the like.

In addition, sodium (E)-4,6-dichloro-3-((2-oxo-1-phenyl-3-pyrrolidinylidene)methyl)-1H-indole-2-carboxylate (GV-196771A) which is one of 4,6-dichloro-1H-indole-2-carboxylic acid derivatives or salts thereof is described in Patent Document 3 as an antagonist of NMDA useful as a therapeutic agent for Alzheimer disease, Huntington disease, amyotrophic lateral sclerosis and the like.

Also in Patent Document 4, 4,6-dichloro-1H-indole-2-carboxylic acid derivative or a salt thereof is described as an indole derivative which has a sphingosine-1-phosphate (hereinafter referred to also as "S1P") receptor agonistic and/or antagonistic activity and is useful as a therapeutic agent for a glaucoma, a dry eye, a vascularization, a cardiovascular disease and the like.

Nevertheless, the preventive or therapeutic effect of a 4,6-dichloro-1H-indole-2-carboxylic acid derivative or a salt thereof on an optic nerve disorder is not described expressly or specifically in these Patent Document.

Patent Document 1: Japanese Patent Laid Open Publication No.6-49027
Patent Document 2: WO 1993/21153
Patent Document 3: WO 1995/10517
Patent Document 4: WO 2007/112322

Summary of the Invention

Problems to be Solved

It is a very interesting objective to discover a compound useful as a preventive or therapeutic agent for an optic nerve disorder, a retinal nerve cell death inhibitor, or a neurofilament light chain expression level recovering agent, especially to discover a compound which exerts said pharmacological effect by an oral administration or an ophthalmic local administration.

Means to Solve the Problems

Inventors investigated an enormous number of compounds for an efficacy in an NMDA-induced rat retinal disorder model for the purpose of discovering a compound useful as a preventive or therapeutic agent for an optic nerve disorder, a retinal nerve cell death inhibitor, or a neurofilament light chain expression level recovering agent. As a result, it was discovered that a 4,6-dichloro-1H-indole-2-carboxylic acid derivative or a salt thereof is:

1) capable, when given orally in an NMDA-induced rat retinal disorder model, of inhibiting a reduction in the cell count in its retinal nerve cell layer;
2) capable, when given intravitreously in an NMDA-induced rat retinal disorder model, of recovery from a reduction in the expression level of the neurofilament light chain (one of major constituents of the optic nerve axon and a component important in maintaining the morphology of the optic nerve axon) in the retina; and,
3) free of, or capable of reducing, a side effect, such as a weight gain inhibiting effect, observed when using other NMDA receptor blockers such as memantine;

thus establishing the present invention.

Thus, the present invention relates to a preventive or therapeutic agent for an optic nerve disorder, especially to a preventive or therapeutic agent which is free of, or capable of reducing, a side effect such as a weight gain inhibiting effect, comprising as an active ingredient at least one of the compound represented by Formula (1):

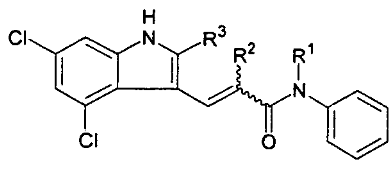

(1)

wherein $R^1$ is a hydrogen atom or a lower alkyl group;
$R^2$ is a hydrogen atom or a lower alkyl group;
$R^1$ and $R^2$ may be joined each other to form an aziridine ring, an azetidine ring, a pyrrolidine ring, a piperidine ring or an azepane ring; and
$R^3$ is a carboxyl group or an ester thereof or an amide thereof, hereinafter being applicable similarly;
or a salt thereof(hereinafter referred to as " the present compound").

The definitions of the terms employed in the specification are described in detail below.

A "halogen atom" refers to a fluorine, chlorine, bromine or iodine atom.

A "lower alkyl group" refers to a straight or branched alkyl group having 1 to 8, preferably 1 to 6, especially 1 to 4 carbon atoms. The typical examples thereof include methyl, ethyl, n-propyl, n-butyl, n-pentyl, n-hexyl, n-heptyl, n-octyl, isopropyl, isobutyl, sec-butyl, tert-butyl, isopentyl groups and the like.

A "halogeno lower alkyl group" refers to a lower alkyl group substituted with one or a plurality of (preferably 2

or 3) halogen atoms. The typical examples thereof include trifluoromethyl, difluoromethyl groups and the like.

An "aryl group" is a residue resulting from removal of one hydrogen atom from a C6-C14 monocyclic aromatic hydrocarbon or dicyclic or tricyclic fused polycyclic aromatic hydrocarbon. The typical examples thereof include phenyl, naphthyl, anthryl, phenanthryl groups and the like.

A "lower alkoxy group" refers to a group resulting from substitution of a hydrogen atom in a hydroxy group with a lower alkyl group. The typical examples thereof include methoxy, ethoxy, n-propoxy, n-butoxy, n-pentoxy, n-hexyloxy, n-heptyloxy, n-octyloxy, isopropoxy, isobutoxy, sec-butoxy, tert-butoxy, isopentoxy groups and the like.

A "halogeno lower alkoxy group" refers to a lower alkoxy group substituted with one or a plurality of (preferably 2 or 3) halogen atoms. The typical examples thereof include trifluoromethoxy, difluoromethoxy groups and the like.

A "lower alkyl group optionally having a substituent" refers to an unsubstituted lower alkyl group and a lower alkyl group having a substituent. The lower alkyl group having a substituent refers to a lower alkyl group substituted with one or a plurality of (preferably 2 or 3) groups selected from the group consisting of a halogen atom, a lower alkyl group, a halogeno lower alkyl group, an aryl group, a lower alkoxy group and a halogeno lower alkoxy group.

An "aryl group optionally having a substituent" refers to an unsubstituted aryl group and a aryl group having a substituent. The aryl group having a substituent refers to an aryl group substituted with one or a plurality of (preferably 2 or 3) groups selected from the group consisting of a halogen atom, a lower alkyl group, a halogeno lower alkyl group, a lower alkoxy group and a halogeno lower alkoxy group.

An "ester of a carboxy group" refers to a group represented by Formula (2):

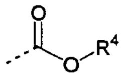 (2)

wherein $R^4$ is a hydrogen atom, a lower alkyl group optionally having a substituent or an aryl group optionally having a substituent.

An "amide of a carboxy group" refers to a group represented by Formula (3):

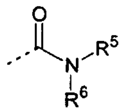 (3)

wherein $R^5$ and $R^6$ are same or different and each is a hydrogen atom, a lower alkyl group optionally having a substituent or an aryl group optionally having a substituent.

As used herein, "plurality" means those which are same to or different from each other, the number of which is preferably 2 or 3, especially 2.

A "salt" in the present compound is not limited particularly as long as it is a pharmaceutically acceptable salt, and may for example be a salt with an inorganic acid such as hydrochloric acid, hydrobromic acid, hydroiodic acid, nitric acid, sulfuric acid, phosphoric acid and the like, a salt with an organic acid such as acetic acid, fumaric acid, maleic acid, succinic acid, citric acid, tartaric acid, adipic acid, gluconic acid, glucoheptoic acid, glucuronic acid, terephthalic acid, methanesulfonic acid, lactic acid, hippuric acid, 1,2-ethanedisulfonic acid, isethionic acid, lactobionic acid, oleic acid, pamoic acid, polygalacturonic acid, stearic acid, tannic acid, trifluoromethanesulfonic acid, benzenesulfonic acid, p-toluenesulfonic acid, lauryl sulfate, methyl sulfate, naphthalenesulfonic acid, sulfosalicylic acid and the like, a quaternary ammonium salt with methyl bromide, methyl iodide and the like, a salt with a halogen ion such as bromine ion, chlorine ion, iodine ion and the like, a salt with an alkaline metal such as lithium, sodium, potassium and the like, a salt with an alkaline earth metal such as calcium, magnesium and the like, a metal salt with iron, zinc and the like, a salt with ammonia, a salt with an organic amine such as triethylenediamine, 2-aminoethanol, 2,2-iminobis(ethanol), 1-deoxy-1-(methylamino)-2-D-sorbitol, 2-amino-2-(hydroxymethyl)-1,3-propanediol, procaine, N,N-bis(phenylmethyl)-1,2-ethanediamine and the like.

When the present compound has a geometric isomer and/or an optical isomer, such an isomer is also encompassed within the scope of the present compound.

When the present compound has a proton tautomerism, its tautomers (keto form, enol form) are also encompassed within the scope of the present compound.

When the present compound has a hydrate and/or a solvate, such a hydrate and/or a solvate is also encompassed within the scope of the present compound.

When the present compound has a polymorphism and a polymorphic group (polymorphic system), its polymorphic form and polymorphic group (polymorphic system) are also encompassed within the scope of the present compound. As used herein, the polymorphic group (polymorphic system) means crystal forms in respective stages over a course of change in the crystal form depending on the condition and the state (which includes a formulated state) of crystal production, precipitation, storage and the like, as well as the entire of such a course.

(a) As an example of the present compound, a compound or a salt thereof in which respective groups in a compound represented by Formula (1) or a salt thereof are the groups shown below can be given.

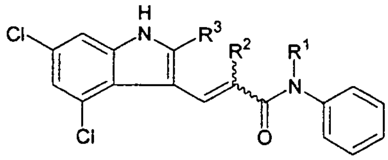
(1)

(a1) $R^1$ is a hydrogen atom or a lower alkyl group; and/or, (a2) $R^2$ is a hydrogen atom or a lower alkyl group; and/or, (a3) $R^1$ and $R^2$ may be joined each other to form an aziridine ring, an azetidine ring, a pyrrolidine ring, a piperidine ring or an azepane ring; and/or,
(a4) $R^3$ is a carboxyl group or an ester thereof or an amide thereof.

Thus, a compound resulting from every combination selected from the abovementioned (1a), (2a), [or (3a)] and (4a) in a compound represented by Formula (1) or a salt thereof. (b) As a preferred example of the present compound, a compound or a salt thereof in which respective groups in a compound represented by Formula (1) or a salt thereof are the groups shown below can be given.
(1b) $R^1$ is a hydrogen atom; and/or,
(2b) $R^2$ is a hydrogen atom; and/or,
(3b) $R^1$ and $R^2$ may be joined each other to form a pyrrolidine ring; and/or,
(4b) $R^3$ is a carboxyl group.

Thus, a compound or a salt thereof resulting from every combination selected from the abovementioned (1b), (2b), [or (3b)] and (4b) in a compound represented by Formula (1) or a salt thereof.

A compound or a salt thereof which meets with the combination of these (b) requirements with the abovementioned (a) requirements is within the scope of the present compound.

As a preferred typical example of the present compound, the following compounds and salts thereof can be given.

· (E)-4,6-dichloro-3-(3-oxo-3-(phenylamino)-1-propenyl)-1H-indole-2-carboxylic acid (Compound A), or

· (E)-4,6-dichloro-3-((2-oxo-1-phenyl-3-pyrrolidinylidene)methyl)-1H-indole-2-carboxylic acid (Compound B).

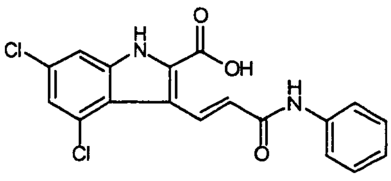
(Compound A)

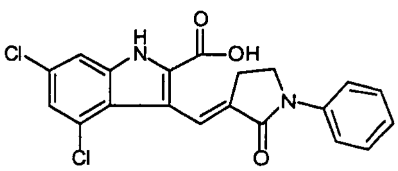
(Compound B)

As an especially preferred typical example of the present compound, the following compounds and salts thereof

can be given.

· Sodium (E)-4,6-dichloro-3-(3-oxo-3-(phenylamino)-1-propenyl)-1H-indole-2-carboxylate (Compound A', GV-150526A), or,

· sodium (E)-4,6-dichloro-3-((2-oxo-1-phenyl-3-pyrrolidinylidene)methyl)-1H-indole-2-carboxylate (Compound B',GV-196771A).

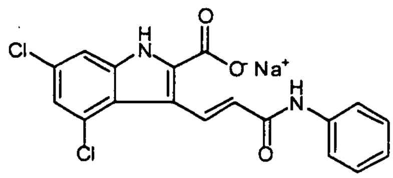

(Compound A')

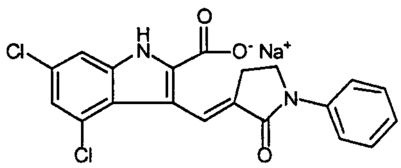

(Compound B')

A preventive or therapeutic agent for an optic nerve disorder, a retinal nerve cell death inhibitor, or a neurofilament light chain expression level recovering agent containing at least one of the present compounds, preferably one of the present compounds as an active ingredient is the present invention.

An "optic nerve disorder" in the invention means an optic nerve disorder and/or an ophthalmic disease accompanied with an optic nerve disorder.

While an "ophthalmic disease accompanied with an optic nerve disorder" as used herein is not limited particularly as long as it is an ophthalmic disease accompanied with an optic nerve disorder and may for example be glaucomatous diseases, retinal diseases, ischemic disorders and the like, the typical examples thereof include glaucoma, glaucomatous constriction of visual fields, glaucomatous optic nerve atrophy, glaucomatous optic neurosis, central retinal artery occlusion, branch retinal arterial occlusion, central retinal vein occlusion, branch retinal venous occlusion, diabetic retinopathy, macular degeneration (age-related macular degeneration such as atrophic age-related macular degeneration, exudative age-related macular degeneration and the like), retinitis pigmentosa, retinopathy of prematurity, retinal detachment, retinal pigment epithelial detachment, Leber disease, ischemic optic neurosis and the like.

While a "glaucomatous optic nerve disorder" in the invention is not limited particularly as long as it is an optic nerve disorder originated from a glaucoma and typically means a glaucoma and/or an ophthalmic disease accompanying a glaucoma, more typical examples thereof include glaucomatous optic nerve disorders such as glaucoma, glaucomatous constriction of visual fields, glaucomatous optic nerve atrophy, glaucomatous optic neurosis and the like.

A "retinal nerve cell" in the invention means a nerve cell involved in the transmission of a visual signal to a brain, and typically means a photoreceptor cell, a horizontal cell, a bipolar cell, an optic ganglion cell, an amacrine cell and the like.

A "retinal nerve cell death" in the invention means an apoptosis and/or a necrosis of a retinal nerve cell.

A "neurofilament light chain expression level recovering agent" in the invention means an agent which inhibits the reduction of and/or increases the expression level of the neurofilament light chain in a retina. This neurofilament light chain is one of major constituents of the optic nerve axon, and is a component important in maintaining the morphology of the optic nerve axon.

This compound can be administered orally or parenterally. The administration mode may for example be an oral administration, an ophthalmic local administration (instillation, administration into conjunctival sac, intravitreous administration, subconjunctival administration, administration into Tenon's capsule and the like), intravenous administration, percutaneous administration and the like, and a pharmaceutically acceptable additive may appropriately be

selected and used if necessary to formulate a dosage form suitable to the administration mode.

The dosage form may for example be a tablet, a capsule, a granule, a powder and the like for an oral formulation and an injection formulation, an eye drop, an ointment, a patch, a gel, an insert and the like for a parenteral formulation.

In case for example of a tablet, a capsule, a granule, a powder and the like, those which can appropriately be selected and used if necessary to formulate a dosage form are excipients such as lactose, glucose, D-mannitol, anhydrous calcium hydrogen phosphate, starch, sucrose and the like; disintegrants such as carboxymethyl cellulose, calcium carboxymethyl cellulose, sodium crosscarmelose, crosspovidon, starch, partially alphatized starch, low-substituted hydroxypropyl cellulose and the like; binders such as hydroxypropyl cellulose, ethyl cellulose, gum arabic, starch, partially alphatized starch, polyvinyl pyrrolidone, polyvinyl alcohol and the like; lubricants such as magnesium stearate, calcium stearate, talc, hydrated silicon dioxide, hardened oil and the like; coatings such as purified sugar, hydroxypropyl methyl cellulose, hydroxypropyl cellulose, methyl cellulose, ethyl cellulose, polyvinyl pyrrolidone and the like; flavors such as citric acid, aspartame, ascorbic acid, menthol and the like.

An injection formulation (aqueous, oily, suspension) can be formulated while selecting and using, appropriately if necessary, an isotonicity-imparting agent such as sodium chloride and the like; a solvent or solubilizing agent such as a soybean oil, Macrogol and the like; a buffering agent such as sodium phosphate and the like; a surfactant such as Polysorbate 80 and the like; a thickening agent such as sodium carmelose, methyl cellulose and the like; an analgesic agent such as benzyl alcohol and the like, and its pH may be any value within a range acceptable for an injection formulation, and is preferably within a range from pH 4 to 8.

An eye drop (aqueous, oily, suspension) can be formulated while selecting and using, appropriately if necessary, a solvent or solubilizing agent such as castor oil, glycerol, alcohol, an ether compound between a polyglycerol and an alcohol and the like; an isotonicity-imparting agent such as sodium chloride, conc. glycerin and the like; a buffering agent such as phosphates, carbonates and the like; a surfactant such as polyoxyethylene sorbitan monooleate, polyoxyl stearate 40, polyoxyethylene hardened castor oil and the like; a stabilizer such as sodium citrate, sodium edetate and the like; a preservative such as benzalkonium chloride, paraben and the like; and, in the case of an eye drop suspension, a dispersant such as a water-soluble polymer and the like, an isotonicity-imparting agent such as sodium chloride, conc.glycerin and the like; a buffering agent such as phosphates, carbonates and the like; a surfactant such as Polysorbate 80, polyoxyl stearate 40, polyoxyethylene hardened castor oil 60 and the like; a stabilizer such as sodium citrate, sodium edetate and the like; a preservative such as benzalkonium chloride, paraben and the like, and its pH may be any value within a range acceptable for an ophthalmologic eye drop formulation, and is preferably within a range from pH 4 to 8.

An ophthalmic ointment can be formulated using a commonly employed base material such as a white petrolatum, a liquid paraffin and the like.

An insert can be formulated using a biodegradable polymer such as hydroxypropyl cellulose, hydroxypropyl methyl cellulose, carboxyvinyl polymer, polyacrylic acid and the like while selecting and using, appropriately if necessary, an excipient, a binder, a stabilizer, a pH modifier and the like.

An intraocular implant formulation can be formulated using a biodegradable polymer such as polylactic acid, polyglycolic acid, lactic acid/glycolic acid copolymer, lactic acid-caprolacton copolymer, polyanhydride, polyorthoester, poly s-caprolacton and the like, while selecting and using, appropriately if necessary, an excipient, a binder, a stabilizer, a pH modifier and the like. Formulation can be accomplished also by using a non-biodegradable polymer such as an ethylene vinyl acetate copolymer and the like, while selecting and using, appropriately if necessary, an excipient, a binder, a stabilizer and the like.

The dose of the present compound can be selected appropriately depending on the dosage form, the condition, age, body weight of the patient and the like. In the case for example of oral administration, a dosage of 0.01 to 5000 mg, preferably 0.1 to 2500 mg, especially 0.5 to 1000 mg can be administered in portions 1 to several times a day. In the case of injection, a dosage of 0.00001 to 2000 mg, preferably 0.0001 to 1500 mg, especially 0.001 to 500 mg can be administered in portions 1 to several times a day. In the case of an eye drop, a dosage of 0.00001 to 10% w/v, preferably 0.0001 to 5% w/v, especially 0.001 to 1% w/v can be administered 1 to several times a day. An eye ointment containing 0.0001 to 2000 mg can be applied. An insert or intraocular implant containing 0.0001 to 2000 mg can be inserted or implanted.

## Advantageous Effects of the Invention

As described below in detail in the section of a pharmacological test, the present compound was capable, when given orally in an NMDA-induced rat retinal disorder model, of inhibiting a reduction in the cell count in its retinal nerve cell layer. Also it was capable, when given intravitreously in the same model, of recovery from a reduction in the expression level of the neurofilament light chain in a retina.

Accordingly, the present compound is useful as a preventive or therapeutic agent for an optic nerve disorder, a retinal nerve cell death inhibitor, or a neurofilament light chain expression level recovering agent, especially useful as a preventive or therapeutic agent for a glaucomatous optic nerve disorder, more typically, a preventive or therapeutic

agent for a glaucomatous ophthalmic disease such as a glaucoma, a glaucomatous constriction of visual fields, a glaucomatous optic nerve atrophy, a glaucomatous optic neurosis and the like, and also useful as a preventive or therapeutic agent which is free of, or capable of reducing, a side effect, such as a weight gain inhibiting effect, observed when using other NMDA receptor blockers such as memantine.

Best Mode for Carrying Out the Invention

The followings are the results of pharmacological tests and the formulation examples according to the invention. These examples are intended to ensure a better understanding of the invention and are not intended to restrict the scope of the invention.

[Pharmacological tests]

Example 1

1. Test using NMDA-induced rat retinal disorder model (Evaluation of retinal section after oral administration of test compound)

A NMDA-induced rat retinal disorder model (Invest. Ophthalmol. Vis. Sci., 2003; 44:385-392) employed widely as an optic nerve disorder model was employed to evaluate the usefulness (retinal nerve cell death inhibiting effect) of the present compound.

(Method for producing NMDA-induced rat retinal disorder model)

A rat [Slc:SD, male, about 7 week-old) was allowed to inhale a gas containing 3 to 4% (v/v) vaporized isoflurane at a rate of 1 to 1.5 L air/min to accomplish a systemic introducing anesthesia, followed by a maintaining anesthesia with 2 to 3% (v/v) isoflurane. Thereafter, 5 μl of a solution of NMDA (Sigma, catalog No.M3262) dissolved at 2 mmol/L in a phosphate buffer (hereinafter referred to also as "PBS") was given intravitreously (10 nmol as NMDA).

(Methods for producing and evaluating retinal section)

The rat 3 days after the intravitreous administration of the NMDA solution was treated with a 100 mg/kg sodium pentobarbital injection solution by an intraperitoneal administration to accomplish a systemic anesthesia and then the eyeballs were enucleated. The enucleated eyeballs were fixed in a 25% (w/v) glutaraldehyde: 10% (w/v) neutral buffered formalin = 1:9 mixture. The fixed eyeballs were embedded with a paraffin and then sliced into retinal sections (3 μm in thickness), which were then subjected to a hematoxylin and eosin staining. Eight retinal sections were taken per eyeball at intervals of 45 μm so that the optic nerve papilla was included. Among these 8 sections, 3 sections were selected randomly, and a selected section was photographed for the retina of 700 to 900 μm in width with its center being located about 1.25 mm from the optic nerve papilla, using which the cell count in the retinal ganglion cell layer (hereinafter referred to also as "GCL cell count") was calculated (average value). From the resultant GCL cell count, a % GCL cell count reduction inhibition of each test substance was calculated according to Equation 1 while assigning 100% to the non-treatment group and 0% to a group of 10 nmol NMDA given intravitreously + base given orally. Each group included 4 animals (7 to 8 eyeballs).

[Equation 1]

$$\%\ \text{GCL cell count reduction inhibition} = (N_X - N_O)/(N_U - N_O) \times 100$$

wherein $N_u$ is a GCL cell count in the non-treatment group, No is a GCL cell count in the group of 10 nmol NMDA given intravitreously + base given orally, and $N_x$ is a GCL cell count in the group of 10 nmol NMDA given intravitreously + a test compound given orally.

(Body weight evaluation method)

Over a period from the day of the intravitreous NMDA administration through the eyeball enucleation, each individual animal was weighed once a day. Based on the body weight of the individual animal on the day of the intravitreous NMDA administration, the difference from the body weight on the day of the eyeball enucleation was calculated and the weight gain of each individual animal was calculated (average value). Each group included 4 animals.

(Administration method)

[Group of NMDA given intravitreously + base given orally]

5 μL of an NMDA solution dissolved in PBS (10 nmol as NMDA) was administered intravitreously. A 0.5% (w/v) aqueous solution of methyl cellulose was given by a repetitive oral administration twice a day in a volume of 10 mL/kg for 3 days from the day of the intravitreous NMDA administration until 2 days later. On the day of the intravitreous NMDA administration, a 0.5% (w/v) aqueous solution of methyl cellulose was given orally two times which were about 1 hour before the intravitreous NMDA administration and 1 to 2 hours.

[Group of NMDA given intravitreously + each test compound given orally]

5 μL of an NMDA solution dissolved in PBS (10 nmol as NMDA) was administered intravitreously. Each test compound suspended in 0.5% (w/v) methyl cellulose solution was given at a dose of 30 mg/kg by a repetitive oral administration twice a day in a volume of 10 mL/kg for 3 days from the day of the intravitreous NMDA administration until 2 days later. On the day of the intravitreous NMDA administration, each test compound suspended in 0.5% (w/v) methyl cellulose solution was given orally at a dose of 30 mg/kg two times which were about 1 hour before the NMDA administration and 1 to 2 hours.

[Group of NMDA given intravitreously + control compound given orally]

As a control compound, memantine was employed. 5 μL of an NMDA solution in PBS (10 nmol as NMDA) was administered intravitreously. Memantine dissolved in 0.5% (w/v) methyl cellulose solution was given at a dose of 30 mg/kg by a repetitive oral administration twice a day in a volume of 10 mL/kg for 3 days from the day of the intravitreous NMDA administration until 2 days later. On the day of the intravitreous NMDA administration, memantine dissolved in 0.5% (w/v) methyl cellulose solution was given orally at a dose of 30 mg/kg two times which were about 1 hour before the NMDA administration and 1 to 2 hours.

(Test results)

The results of the test using Compound A' and Compound B' as test compounds were shown in Table 1. As evident from Table 1, Compound A', Compound B' and a control compound memantine inhibited the reduction in the GCL cell count in the retina which is observed usually in the NMDA-induced rat retinal disorder model.

[Table 1]

| Groups | % Inhibition of GLC cell count reduction |
|---|---|
| Group of NMDA given intravitreously + Compound A' given orally | 17.0 |
| Group of NMDA given intravitreously + Compound B' given orally | 14.8 |
| Group of NMDA given intravitreously + memantine given orally | 20.7 |

The results of the weight gain test are shown in Table 2. As evident from Table 2, Compound A' and Compound B'

allowed for a weight gain almost equivalent to that in the non-treatment group, while memantine inhibited the weight gain when compared with the non-treatment group.

[Table 2]

| Groups | Weight gain (average) |
|---|---|
| Non-treatment group | 14.1 |
| Group of NMDA given intravitreously + base given orally | 16.6 |
| Group of NMDA given intravitreously + Compound A' given orally | 12.6 |
| Group of NMDA given intravitreously + Compound B' given orally | 14.0 |
| Group of NMDA given intravitreously + memantine given orally | -3.6 |

(Discussion)

Based on the results of the test, the present compounds including Compound A' and Compound B' exhibited a preventive or therapeutic effect on the optic nerve disorders and a retinal nerve cell death inhibiting effect without causing the weight gain inhibition as a side effect when given in the administration mode for a systemic exposure such as an oral administration. They have a preventive or therapeutic effect especially on glaucomatous optic nerve disorders. On the other hand, memantine caused the weight gain inhibition as a side effect although it had similar preventive or therapeutic effect on the optic nerve disorders and retinal nerve cell death inhibiting effect, and accordingly the present compounds are suggested to be more useful in terms of the inhibiting effect described above.

Example 2

2. Test using NMDA-induced rat retinal disorder model (Quantitative polymerase chain reaction (hereinafter referred to also as "PCR") evaluation after intravitreous administration of test compounds)

(Quantitative PCR evaluation method)

In the NMDA-induced rat retinal disorder model, the efficacy of the test compounds were evaluated using as an index the expression level in the retinal tissue of the neurofilament light chain (hereinafter referred to also as "NFL") which is one of major constituents of the optic nerve axon and is considered to be important in maintaining the morphology of the optic nerve axon.

(Evaluation method)

The rat 1 day after the intravitreous administration of NMDA was treated with a 100 mg/kg sodium pentobarbital injection solution by an intraperitoneal administration to accomplish a systemic anesthesia and then the eyeballs were enucleated, and the retina was isolated. The isolated retina was homogenized, and a total RNA was extracted using QIAzol Lysis Reagent (QIAGEN, catalog No.79306) and RNeasy 96 Kit (250) (QIAGEN, catalog No.74106), and then cDNA was synthesized using PrimeScript (trademark) RT reagent Kit (TAKARA, catalog No.RR037B). In accordance with the instruction attached to QuantiTect Multiplex PCR Kit (1000) (QIAGEN, catalog No.204545), QuantiTect Multiplex PCR Master Mix, the synthesized cDNA, the primers/probe for NFL (Sigma, "Ordermade") and the primers/probe for glycerylaldehyde 3-phosphate dehydrogenase (hereinafter referred to also as "GAPDH") (Applied Biosystems, Catalog No.4352338E) were mixed, and a quantitative PCR machine (Applied Biosystems, Catalog No.7500-03) was employed to conduct a PCR reaction, whereby measuring the expression levels of NFL and GAPDH.

From the NFL and GAPDH expression levels thus obtained, a relative NFL expression level was calculated by correcting the NFL expression level based on the housekeeping gene GAPDH expression level in accordance with Equation 2. Thereafter, a % NFL recovery of the test compound was calculated based on the NMDA-induced NFL reduction according to Equation 3 while assigning 100% to the non-treatment group and 0% to the 10 nmol intravitreous NMDA group. Each group included 1 animal (2 eyeballs).

The sequences of the primers and the probe for NFL employed in the invention are shown below.

[Table 3]

| Name | Base sequence |
|---|---|
| NFL Primer 1 | ACAAGCAGAATGCAGACATCA |

(continued)

| Name | Base sequence |
|---|---|
| NFL Primer 2 | GGAGGTCCTGGTACTCCTTC |
| NFL Probe | CCATCTCGCTCTTCGTGCTTCGC |

[Equation 2]

$$\% \text{ Relative NFL expression level} = (\text{NFL expression level}/\text{GAPDH expression level})$$

[Equation 3]

$$\% \text{ NFL recovery} = (E_X - E_O)/(E_P - E_O) \times 100$$

wherein $E_P$ is a relative NFL expression level in the non-treatment group, $E_O$ is a relative NFL expression level in the group of 10 nmol intravitreous NMDA administration and $E_X$ is a relative NFL expression level in the group of intravitreous administration of 10 nmol NMDA mixed with each compound.

(Administration method)

[Group of intravitreous NMDA administration]

5 μL of an NMDA solution dissolved in a 30% (v/v) DMSO-containing purified water (10 nmol as NMDA) was administered intravitreously.

[Group of intravitreous administration of each compound]

5 μL of a mixture solution of NMDA and a test compound dissolved in a 30% (v/v) DMSO-containing purified water (10 nmol as NMDA, 15 nmol as a test compound) was administered intravitreously.

(Test results)

The results of the test using Compound A' and Compound B' as test compounds were shown in Table 4. As evident from Table 4, Compound A' and Compound B' recovered the NFL expression level from the reduction occurring in the NMDA-induced rat retinal disorder model. On the other hand, memantine at the same dose resulted in a recovery from the reduction in the NFL expression level, but its potency was markedly lower than those observed with Compound A' and Compound B'.

[Table 4]

| Group | % NFL Recovery (%, average) |
|---|---|
| Group of intravitreous Compound A' administration, 15 nmol/eye | 86.6 |
| Group of intravitreous Compound B' administration, 15 nmol/eye | 80.7 |
| Group of intravitreous memantine administration, 15 nmol/eye | 33.6 |

(Discussion)

Based on the results of the test, the present compounds including Compound A' and Compound B' exhibited a preventive or therapeutic effect on the optic nerve disorders and an NFL expression level recovering effect also when given in the mode for a local administration. On the other hand, memantine had a preventive or therapeutic effect on

the optic nerve disorders and retinal nerve cell death inhibiting effect, but the local administration of the same dose gave an efficacy which was markedly weaker than those of Compound A' or Compound B', suggesting that the present compounds were stronger in terms of the potential possessed by the compounds themselves.

[Formulation Examples]

Representative formulations of the invention are described below.

Example 3

1) Tablet (in 150 mg)

| | |
|---|---|
| The present compound | 10 mg |
| Lactose | 90 mg |
| Corn starch | 40 mg |
| Calcium carboxymethyl cellulose | 5.5 mg |
| Hydroxypropyl cellulose | 4 mg |
| Magnesium stearate | 0.5 mg |

A tablet formulated as shown above was coated with 3 mg of a coating (for example, a coating such as hydroxypropyl methyl cellulose, Macrogol, talc, titanium oxide, silicone resin and the like) to obtain an intended tablet. Alternatively, the types and/or the amounts of the present compound and the additives may appropriately be varied to obtain a desired tablet.

Example 4

2) Capsule (in 150 mg)

| | |
|---|---|
| The present compound | 50 mg |
| Lactose | 90 mg |
| Calcium carboxymethyl cellulose | 4.5 mg |
| Hydroxypropyl cellulose | 4 mg |
| Magnesium stearate | 1.5 mg |

The types and/or the amounts of the present compound and the additives may appropriately be varied to obtain a desired capsule.

Example 5

3) Water soluble eye drop (in 100 ml)

| | |
|---|---|
| The present compound | 100 mg |
| 1-O-n-Dodecyl-3-O-methyl-2-O-2',3'-dihydroxypropyl glycerol | 7000 mg |
| Ethanol | 5 mL |
| Sodium chloride | 900 mg |
| Sterilized purified water | q.s. |

The types and/or the amounts of the present compound and the additives may appropriately be varied to obtain a desired aqueous eye drop.

Example 6

4) Oily eye drop (in 100 ml)

| | |
|---|---|
| The present compound | 100 mg |
| Castor oil | q.s. |

The types and/or the amounts of the present compound and the additives may appropriately be varied to obtain a desired oily eye drop.

Example 7

5) Eye drop suspension (in 100 ml)

| | |
|---|---|
| The present compound | 100 mg |
| Sodium chloride | 750 mg |
| Benzalkonium chloride | 5 mg |
| Polysorbate 80 | q.s. |
| Hydroxypropylmethyl cellulose | q.s. |
| Sodium edetate | q.s. |
| Sodium hydrogen phosphate | q.s. |
| Sodium dihydrogen phosphate | q.s. |
| Sterilized purified water | q.s. |

The types and/or the amounts of the present compound and the additives may appropriately be varied to obtain a desired eye drop suspension.

Example 8

6) Aqueous solution for injection (in 100 ml)

| | |
|---|---|
| The present compound | 100 mg |
| Sodium chloride | 750 mg |
| Macrogol 400 | 1000 mg |
| Sodium hydroxide | q.s. |
| Hydrochloric acid | q.s. |
| Sterilized purified water | q.s. |

The types and/or the amounts of the present compound and the additives may appropriately be varied to obtain a desired aqueous solution for injection.

Example 9

7) Oil for injection (in 100 ml)

| | |
|---|---|
| The present compound | 100 mg |
| Soybean oil | q.s. |

The types and/or the amounts of the present compound and the additives may appropriately be varied to obtain a desired oil for injection.

Example 10

8) Suspension for injection (in 100 ml)

| | |
|---|---|
| The present compound | 100 mg |
| Sodium chloride | 750 mg |
| Sodium carmelose | 750 mg |
| Polysorbate 80 | 40 mg |
| Benzyl alcohol | 40 mg |
| Sodium hydroxide | q.s. |
| Hydrochloric acid | q.s. |
| Sterilized purified water | q.s. |

The types and/or the amounts of the present compound and the additives may appropriately be varied to obtain a desired suspension for injection.

Industrial Applicability

The present compound is useful as a preventive or therapeutic agent for an optic nerve disorder, a retinal nerve cell death inhibitor, or a neurofilament light chain expression level recovering agent, especially useful as a preventive or therapeutic agent for a glaucomatous optic nerve disorder, more typically, a preventive or therapeutic agent for a glaucomatous ophthalmic disease such as a glaucoma, a glaucomatous constriction of visual fields, a glaucomatous optic nerve atrophy, a glaucomatous optic neurosis and the like.

## Claims

1. A preventive or therapeutic agent for an optic nerve disorder comprising as an active ingredient at least one of the compound represented by Formula (1):

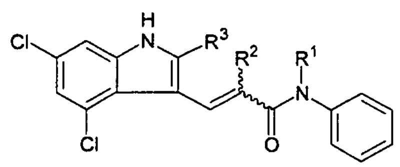
(1)

wherein $R^1$ is a hydrogen atom or a lower alkyl group;
$R^2$ is a hydrogen atom or a lower alkyl group;
$R^1$ and $R^2$ may be joined each other to form an aziridine ring, an azetidine ring, a pyrrolidine ring, a piperidine ring or an azepane ring; and
$R^3$ is a carboxyl group or an ester thereof or an amide thereof;
or a salt thereof.

2. The preventive or therapeutic agent according to Claim 1 wherein in Formula (1):

$R^1$ is a hydrogen atom;
$R^2$ is a hydrogen atom;
$R^1$ and $R^2$ may be joined each other to form a pyrrolidine ring; and
$R^3$ is a carboxyl group.

3. The preventive or therapeutic agent according to Claim 2 wherein the compound represented by Formula (1) is:

· (E)-4,6-dichloro-3-(3-oxo-3-(phenylamino)-1-propenyl)-1H-indole-2-carboxylic acid, or
· (E)-4,6-dichloro-3-((2-oxo-1-phenyl-3-pyrrolidinylidene)methyl)-1H-indole-2-carboxylic acid.

4. A preventive or therapeutic agent for an optic nerve disorder comprising as an active ingredient sodium (E)-4,6-dichloro-3-(3-oxo-3-(phenylamino)-1-propenyl)-1H-indole-2-carboxylate (GV-150526A).

5. A preventive or therapeutic agent for an optic nerve disorder comprising as an active ingredient sodium (E)-4,6-dichloro-3-((2-oxo-1-phenyl-3-pyrrolidinylidene)methyl)-1H-indole-2-carboxylate (GV-196771A).

6. The preventive or therapeutic agent according to any one of Claims 1 to 5 wherein the optic nerve disorder is a glaucomatous optic nerve disorder.

7. The preventive or therapeutic agent according to Claim 6 wherein the glaucomatous optic nerve disorder is a glaucoma, a glaucomatous constriction of visual fields, a glaucomatous optic nerve atrophy or a glaucomatous optic neurosis.

8. The preventive or therapeutic agent according to any one of Claims 1 to 5 wherein the optic nerve disorder is an axonal transport disorder of a retinal ganglion cell.

9. An inhibitor of a retinal nerve cell death comprising as an active ingredient at least one of the compound according to any one of Claims 1 to 5 or a salt thereof.

10. The inhibitor according to Claim 9 wherein the retinal nerve cell is a retinal ganglion cell.

11. A neurofilament light chain expression level recovering agent comprising as an active ingredient at least one of the compound according to any one of Claims 1 to 5 or a salt thereof.

12. The preventive or therapeutic agent according to any one of Claims 1 to 7 wherein the efficacy is exerted by an oral administration.

13. The preventive or therapeutic agent according to any one of Claims 1 to 7 wherein the efficacy is exerted by an ophthalmic local administration.

14. A method for preventing or treating an optic nerve disorder comprising administering to a patient a pharmacologically effective amount of at least one of the compound represented by Formula (1):

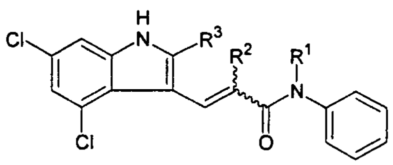 (1)

wherein $R^1$ is a hydrogen atom or a lower alkyl group; $R^2$ is a hydrogen atom or a lower alkyl group;
$R^1$ and $R^2$ may be joined each other to form an aziridine ring, an azetidine ring, a pyrrolidine ring, a piperidine ring or an azepane ring; and
$R^3$ is a carboxyl group or an ester thereof or an amide thereof;
or a salt thereof.

15. The method according to Claim 14 wherein in Formula (1) :

$R^1$ is a hydrogen atom;
$R^2$ is a hydrogen atom;
$R^1$ and $R^2$ may be joined each other to form a pyrrolidine ring; and
$R^3$ is a carboxyl group.

**16.** The method according to Claim 14 wherein the compound represented by Formula (1) is:

· (E)-4,6-dichloro-3-(3-oxo-3-(phenylamino)-1-propenyl)-1H-indole-2-carboxylic acid, or
· (E)-4,6-dichloro-3-((2-oxo-1-phenyl-3-pyrrolidinylidene)methyl)-1H-indole-2-carboxylic acid.

**17.** A method for preventing or treating an optic nerve disorder comprising administering to a patient a pharmacologically effective amount of sodium (E)-4,6-dichloro-3-(3-oxo-3-(phenylamino)-1-propenyl)-1H-indole-2-carboxylate (GV-150526A).

**18.** A method for preventing or treating an optic nerve disorder comprising administering to a patient a pharmacologically effective amount of sodium (E)-4,6-dichloro-3-((2-oxo-1-phenyl-3-pyrrolidinylidene)methyl)-1H-indole-2-carboxylate (GV-196771A).

**19.** The method according to any one of Claims 14 to 18 wherein the optic nerve disorder is a glaucomatous optic nerve disorder.

**20.** The method according to Claim 19 wherein the glaucomatous optic nerve disorder is a glaucoma, a glaucomatous constriction of visual fields, a glaucomatous optic nerve atrophy or a glaucomatous optic neurosis.

**21.** The method according to any one of Claims 14 to 18 wherein the optic nerve disorder is an axonal transport disorder of a retinal ganglion cell.

**22.** A method for inhibiting a retinal nerve cell death comprising administering to a patient a pharmacologically effective amount of at least one of the compound represented by Formula (1):

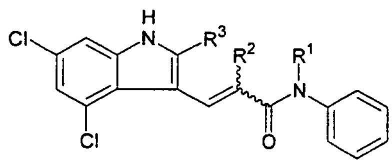

(1)

wherein $R^1$ is a hydrogen atom or a lower alkyl group;
$R^2$ is a hydrogen atom or a lower alkyl group;
$R^1$ and $R^2$ may be joined each other to form an aziridine ring, an azetidine ring, a pyrrolidine ring, a piperidine ring or an azepane ring; and
$R^3$ is a carboxyl group or an ester thereof or an amide thereof;
or a salt thereof.

**23.** The method according to Claim 22 wherein the retinal nerve cell is a retinal ganglion cell.

**24.** A method for recovering neurofilament light chain expression level comprising administering to a patient a pharmacologically effective amount of at least one of the compound represented by Formula (1):

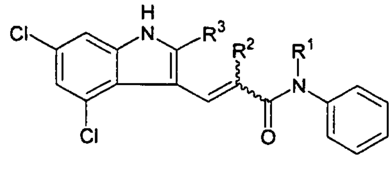

(1)

wherein $R^1$ is a hydrogen atom or a lower alkyl group; $R^2$ is a hydrogen atom or a lower alkyl group;
$R^1$ and $R^2$ may be joined each other to form an aziridine ring, an azetidine ring, a pyrrolidine ring, a piperidine ring or an azepane ring; and
$R^3$ is a carboxyl group or an ester thereof or an amide thereof;
or a salt thereof.

**25.** The method according to any one of Claims 14, 22 and 24 wherein the administration is an oral administration.

**26.** The method according to any one of Claims 14, 22 and 24 wherein the administration is an ophthalmic local administration.

**INTERNATIONAL SEARCH REPORT**

International application No.
PCT/JP2010/053940

A. CLASSIFICATION OF SUBJECT MATTER
*C07D209/42*(2006.01)i, *A61K31/405*(2006.01)i, *A61P27/02*(2006.01)i, *C07D403/06*(2006.01)i

According to International Patent Classification (IPC) or to both national classification and IPC

B. FIELDS SEARCHED

Minimum documentation searched (classification system followed by classification symbols)
C07D209/42, A61K31/405, A61P27/02, C07D403/06

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2010 |
| Kokai Jitsuyo Shinan Koho | 1971-2010 | Toroku Jitsuyo Shinan Koho | 1994-2010 |

Electronic data base consulted during the international search (name of data base and, where practicable, search terms used)
JSTPlus/JMEDPlus/JST7580(JDreamII)

C. DOCUMENTS CONSIDERED TO BE RELEVANT

| Category* | Citation of document, with indication, where appropriate, of the relevant passages | Relevant to claim No. |
|---|---|---|
| Y | JP 06-049027 A (Glaxo S.p.A.), 22 February 1994 (22.02.1994), claim 6; paragraphs [0011] to [0017], [0058]; examples & EP 568136 A1 & WO 93/21153 A1 & US 5373018 A & US 5374648 A & US 5374649 A & JP 07-505407 A & US 5510367 A | 1-13 |
| Y | JP 09-503770 A (Glaxo Wellcome S.p.A.), 15 April 1997 (15.04.1997), claim 12; page 14, 7th line from the bottom to page 16, line 12; examples & WO 95/10517 A1 & EP 723541 A1 & US 5760059 A & US 5962496 A & US 6100289 A | 1-13 |

☒ Further documents are listed in the continuation of Box C.   ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 10 May, 2010 (10.05.10) | 18 May, 2010 (18.05.10) |

| Name and mailing address of the ISA/ | Authorized officer |
|---|---|
| Japanese Patent Office | |
| Facsimile No. | Telephone No. |

Form PCT/ISA/210 (second sheet) (July 2009)

INTERNATIONAL SEARCH REPORT

International application No. PCT/JP2010/053940

C (Continuation). DOCUMENTS CONSIDERED TO BE RELEVANT

| Category* | Citation of document, with indication, where appropriate, of the relevant passages | Relevant to claim No. |
|---|---|---|
| Y | JP 08-506807 A (Massachusetts Eye and Ear Infirmary), 23 July 1996 (23.07.1996), page 6, lines 6 to 21; page 12, lines 2 to 3; page 14, table, lower right & WO 94/13275 A1 & EP 671910 A1 & US 5922773 A & EP 671910 B1 & JP 2006-070039 A | 1-13 |
| Y | Hiroshi KATSUKI et al., "Naizaisei Glycine Ketsugo Bui Ligand ni yoru NMDA Yuhatsu Momaku Shogai no Seigyo", Japanese Journal of Ocular harmacology, 2007, vol.21, no.1, pages 41 to 43, page 41, lower left column, 1st paragraph, page 42, result 1. | 1-13 |
| Y | TYAN, S.H. et al., A novel NMDA receptor antagonist protects against N-methyl-D-aspartate- and glutamate-induced neurotoxicity in the goldfish retina, Eur. J. Pharmacol., 1997, Vol.321, No.2, p.171-179, page 177, item 3.3 | 1-13 |
| Y | WO 2006/123675 A1 (Santen Pharmaceutical Co., Ltd.), 23 November 2006 (23.11.2006), paragraphs [0004], [0008] & JP 2006-348024 A & EP 1884237 A1 & US 2009/088472 A1 & EP 2119440 A1 & EP 2143431 A1 | 1-13 |

Form PCT/ISA/210 (continuation of second sheet) (July 2009)

**INTERNATIONAL SEARCH REPORT**

International application No.
PCT/JP2010/053940

**Box No. II Observations where certain claims were found unsearchable (Continuation of item 2 of first sheet)**

This international search report has not been established in respect of certain claims under Article 17(2)(a) for the following reasons:

1. ☒ Claims Nos.: 14-26
because they relate to subject matter not required to be searched by this Authority, namely:
Claims 14 to 26 include the methods for treatment of the human body or animal body by surgery or therapy and thus relate to a subject matter which this International Searching Authority is not required to carry out a search under
(Continued to extra sheet)

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

1. ☐ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest**

☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet (2)) (July 2009)

**INTERNATIONAL SEARCH REPORT**

International application No.
PCT/JP2010/053940

Continuation of Box No.II-1 of continuation of first sheet(2)

the provision of PCT Rule 67.1(iv) that PCT Rule 43bis.1(b) applies mutatis mutandis.

Form PCT/ISA/210 (extra sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 6049027 A
- WO 199321153 A
- WO 199510517 A
- WO 2007112322 A


### Non-patent literature cited in the description


- *Invest. Ophthalmol. Vis. Sci.,* 2003, vol. 44, 385-392